(19) Europäisches Patentamt
European Patent Office
Office européen des brevets

(11) Veröffentlichungsnummer: **0 274 030 B1**

# (12) EUROPÄISCHE PATENTSCHRIFT

(45) Veröffentlichungstag der Patentschrift: **12.06.91**

(51) Int. Cl.5: **C07D 303/32**, A01N 43/20

(21) Anmeldenummer: **87117030.4**

(22) Anmeldetag: **19.11.87**

## (54) Periplanon A, Verfahren zur Herstellung und seine Verwendung zur Bekämpfung von Schaben.

(30) Priorität: **22.11.86 DE 3639941**

(43) Veröffentlichungstag der Anmeldung:
**13.07.88 Patentblatt 88/28**

(45) Bekanntmachung des Hinweises auf die Patenterteilung:
**12.06.91 Patentblatt 91/24**

(84) Benannte Vertragsstaaten:
**BE CH DE ES FR GB GR IT LI NL**

(56) Entgegenhaltungen:

**CHEMICAL ABSTRACTS, Band 90, Nr. 11, 12. März 1979, Seite 321, 322, Zusammenfassung Nr. 83843j, Columbus, Ohio, US; E. TALMAN et al.: "Sex pheromones of the American cockroach, Periplaneta americana", & ISR. J. CHEM. 1978, 17(3), 227-35**

**DISSERTATION ABSTRACTS INTERNATIONAL, Band 46, Nr. 8, Februar 1986, Seite 2671-B-2672b, Ann Arbor, Mich., US; J. S. SAWYER: "Part I. Alkoxy stabilization and synthetic utility of tetrahedral carbanions. Part II. Synthetic approaches to periplanone A"**

**HETEROCYCLES, Band 25, Januar 1987, Seiten 305-313, Sendai, JP; T.L. Mac DONALD et al.: "Synthetic studies of periplanone A, a sex pheromone of periplaneta americana"**

(73) Patentinhaber: **BASF Aktiengesellschaft**
**Carl-Bosch-Strasse 38**
**W-6700 Ludwigshafen(DE)**

(72) Erfinder: **Hofmeister, Peter, Dr.**
**Bernard-Humblot-Strasse 12**
**W-6730 Neustadt(DE)**
Erfinder: **Krieg, Wolfgang, Dr.**
**Saarstrasse 17**
**W-6721 Weingarten(DE)**
Erfinder: **Neudert, Reinhard, Dr.**
**Südring 35**
**W-6719 Bissersheim(DE)**
Erfinder: **Hauptmann, Hagen, Dr.**
**Kumpfmühlerstrasse 63**
**W-8400 Regensburg(DE)**

EP 0 274 030 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Der Sexuallockstoff der Amerikanischen Schabe (Periplaneta americana) wurde 1952 entdeckt (L.M. Roth, E.R. Willis, Am. Midl. Nat., 47. 66 (1952)).

Arbeiten zur Identifizierung des Lockstoffgemisches führten zu zwei biologisch aktiven Verbindungen, die als Periplanon A und Periplanon B bezeichnet worden sind und denen die nachstehenden Stukturen (VI) und (VII) zugeordnet wurden:

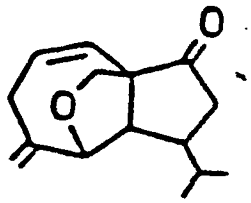

(VI)

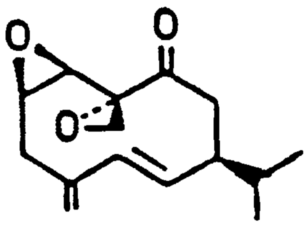

(VII)

(c.J. Persoons et al., [sr. J. Chem. 17, 227 (1978); C.J. Persoons, Dissertation, Wageningen [Niederlande). 1977; C. J. Persoons et al., Tetrahedron Lett., 1976. 2056; W.C. Still. JACS. 101. 2493 (1979)).

Es wurde nun bei dem vergeblichen Versuch. VI zu synthetisieren, gefunden, daß der vermutete Stoff eine andere Struktur haben muß. Im Ergebnis wurde festgestellt, daß ein isomerer Stoff mit anderer Struktur als Sexuallockstoff wirkt. Im Ergebnis wurde festgestellt, daß dieser ebenfalls Periplanon A genannte Wirkstoff (der ursprünglich isolierte wird zur Unterscheidung neuerdings als Isoperiplanon A bezeichnet) nicht eine Struktur besitzt, die mit Formel VI beschrieben werden kann, sondern vielmehr eine dem Periplanon B (Formel VII) ähnliche Struktur der Formel I.

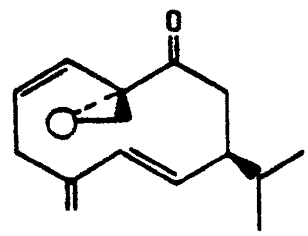

(I)

Gegenüber dem naturlichen Wirkstoff weist der synthetische zwar eine etwas geringere biologische Wirkung auf: vermutlich beruht dies auf der Anwesenheit des sterischen Antipoden - angesichts der vorteilhaften Herstellmethode für das Gemisch der Stereoisomeren ist dies jedoch nicht von Nachteil.

Der hier für (+)-Periplanon A beschriebene Syntheseweg geht von dem bekannten Cyclodecatrienon (II) (W.C. Still. J. Amer. Chem. Soc., 101. 2493 (1979) aus und führt über die neuen Zwischenprodukte. (III).- (IV) und (V) . Die Vorstufe V verdient besonderes Interesse, da sie sich stereoselektiv und mit guter Ausbeute zu (±)-Periplanon A epoxidieren läßt: im einzelnen geht man wie folgt vor:

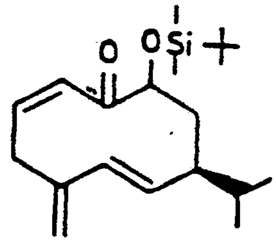

(II)

In einer Olefinierungsreaktion, z.B. mit dem Tebbe-Reagens (F.N. Tebbe et al., J. Am. Chem. Soc.. 100.

3611 (1978)), wird ausgehend von dem Cyclodecatrienon (II) das Cyclodecatetraen III gebildet.

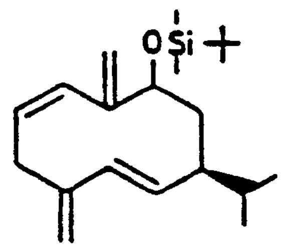

(III)

Durch Abspaltung des t-Butyldimethylsilylrestes wird der Alkohol (IV) mit üblichen Abspaltungsreagenzien für Silylether, vorzugsweise mit Flouriden erhalten: hieraus entsteht durch Oxidation das Cyclodecatetraenon (V).

(IV)

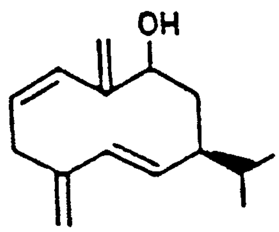

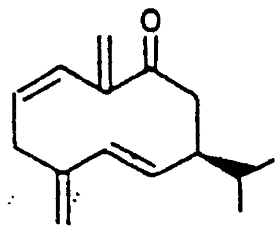

(V)

Als Oxidationsmittel können bekannte Reagenzien dienen und bekannte Verfahren Anwendung finden. Mit guter Ausbeute verläuft die Reaktion bei Verwendung von Pyridiniumchlorchromat auf neutralem Aluminiumoxid (Y.S. Cheng et al., Synthesis, 1980, 223).

Mit Hilfe von Epoxidierungsmitteln kann (V) in (I) überführt werden. Das Reagens t-Butylhydroperoxid/KH beispielsweise epoxidiert (V) in einem Temperaturbereich von 0 bis -50° C, vorzugsweise bei -20° C, stereoselektiv zum Racemat der Struktur I.

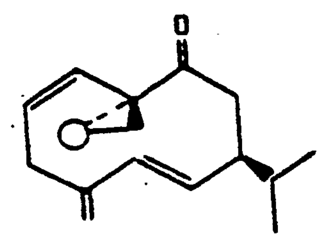

(I)

Die entsprechenden Diastereomeren werden praktisch nicht gebildet.

Das Verfahren wird durch die nachstehenden praktischen Angaben erläutert:

Darstellung von Verbindung III

Eine Lösung von 326 mg (1,14 mmol) Tebbe-Reagens $Cp_2TiCH_2AlCl(CH_3)_2$ in 5 ml Tetrahydrofuran und 230 $\mu$l Pyridin wird bei -40° C mit 255 mg (0,76 mmol) Cyclodecatrienon II, in 1 ml THF gelöst, versetzt. Nach 10 min rührt man noch 40 min bei Raumtemperatur. Anschließend tropft man bei -10° C 2,2 g 15 %ige Natronlauge zu und läßt innerhalb von 15 min auf Raumtemperatur kommen. Man verdünnt mit Diethylether und filtriert durch Kieselgel. Nach Abziehen des Lösungsmittels und säulenchromatographischer Aufarbeitung an Kieselgel mit Petrolether 40/60 als Eluent werden 113 mg (45 %) Cyclodecatetraen III als farbloses Öl isoliert ($R_f$ = 0,3).

Darstellung von Verbindung IV

Man versetzt bei Raumtemperatur eine Lösung von 127 mg (0,38 mmol) der Verbindung III in 1,5 ml Tetrahydrofuran mit 4 ml einer 1-molaren Lösung von Tetrabutylammoniumfluorid in Tetrahydrofuran. Nach 20 min gießt man auf gesättigte Kochsalzlösung, schüttelt dreimal mit Diethylether aus und trocknet über Natriumsulfat. Nach Abziehen des Ethers und säulenchromatographischer Reinigung an Kieselgel mit 20 % t-Butylmethylether in Petrolether (40/60) als Eluent werden 80 mg (96 % Ausb.) IV als farbloses Öl isoliert ($R_f$ = 0,57 (30 % TBME in Petrolether)).

Darstellung von Verbindung V

71 mg (0.33 mmol) des Alkohols IV n 10 ml Diethylether werden bei Raumtemperatur mit 6.0 g Pyridiniumchlorchromat auf neutralem Aluminiumoxid und 1.5 g Natriumhydrogencarbonat 50 min lang geruhrt. Das eingeengte Filtrat liefert nach säulenchromatographischer Reinigung an Kieselgel mit 10 % t-Butylmethylether in Petrolether (40/60) 46 mg (66 % Ausb. ) des Ketons V als farbloses Öl (Rf = 0.61).

Darstellung von Verbindung 1

14 mg (0.35 mmol) Kaliumhydrid werden in 4 ml Tetrahydrofuran suspendiert und bei 0 °C mit 150 µl (0.45 mmol) einer 3-molaren Lösung von t-Butylhydroperoxid in Toluol versetzt. Nach 10 min werden zu dem auf -20 °C abgekühlten Ansatz 40 mg (0, 18 mmol) des Cyclodecatetraenons V in 1 ml Tetrahydrofuran getropft. Man gließt nach 20 min auf Wasser. Schüttelt mit Petrolether aus und trocknet über Natriumsulfat. Die Säulenchromatographie des Rohmaterials an Kieselgel mit 10 % tert.-Butylmethylether in Petrolether (40/60) liefert 290 mg (67 % Ausb.) (±)-Periplanon A als farbloses Öl (Rf = 0.5).

## Ansprüche

1. Racemisches (±)-Periplanon A der Formel I

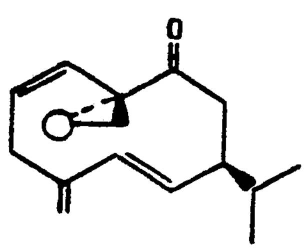

(I)

2. Verfahren zur Herstellung von racemischem Periplanon A der Formel I gemäß Anspruch 1, dadurch gekennzeichnet, daß man ausgehend von dem Cyclodecatrienon der Formel II

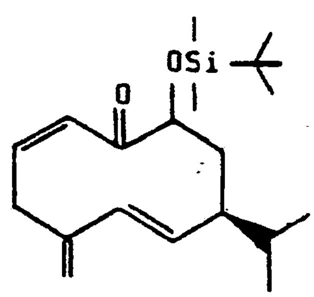

(II)

durch Olefinierung das Cyclodecatetraen der Formel III erzeugt

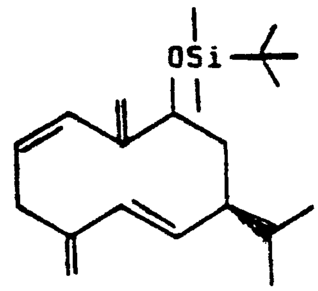

(III)

nach Abspaltung des t-Butyldimethylsilylrestes zum Alkohol der Formel IV diesen durch Oxidation in das Cyclodecatetraenon der Formel V überführt,

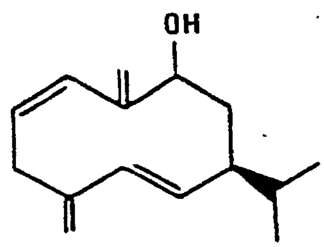

(IV)

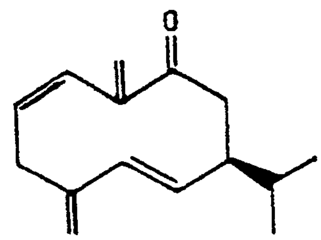

(V)

und epoxidiert.

3. Mittel zur Bekämpfung von Schaben, vorzugsweise der Familien Periplaneta und Blatta, enthaltend Periplanon A der Struktur 1 gemäß Anspruch 1 und gegebenenfalls übliche Zusatzstoffe und Hilfsmittel.

4. Verfahren zur Bekämpfung von Schaben, vorzugsweise der Familien Blatta und Periplaneta, dadurch gekennzeichnet, daß man ein Mittel nach Anspruch 3 in Verbindung mit einem Insektizid anwendet.

5. Verfahren zur Beeinflussung der Vermehrungsrate von Schaben, vorzugsweise der Familie Blatta und Periplaneta, dadurch gekennzeichnet, daß man ein Mittel nach Anspruch 3 in einer solchen Menge anwendet, daß die männlichen Tiere der Art einer Familie bei der Auffindung der weiblichen Tiere gestört werden.

## Claims

1. Racemic (±)-periplanone A of the formula I

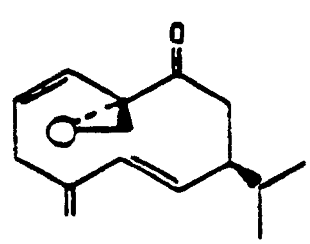

(I)

2. A process for the preparation of racemic periplanone A of the formula I as claimed in claim 1, wherein the cyclodecatrienone of the formula II

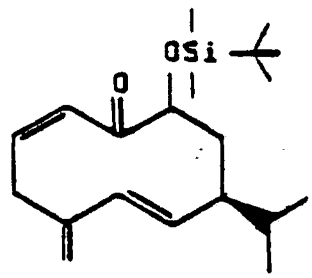

(II)

is olefinated to produce the cyclodecatetraene of the formula III

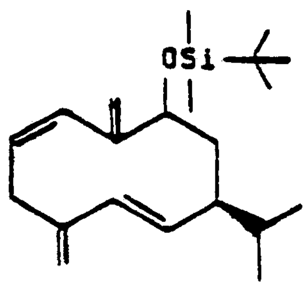

(III)

the tert-butyldimethylsilyl radical is eliminated to give the alcohol of the formula IV and the latter is converted to the cyclodecatetraenone of the formula V by oxidation

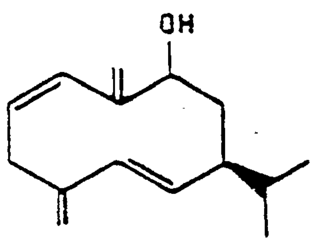

(IV)

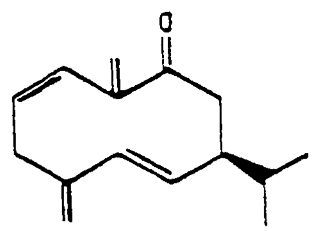

(V)

and then epoxidized.

3. An agent for controlling cockroaches, preferably those of the families Periplaneta and Blatta, containing periplanone A of the structure I as claimed in claim 1 with or without conventional additives and assistants.

4. A method of controlling cockroaches, preferably those of the families Blatta and Periplaneta, wherein an agent as claimed in claim 3 is used in conjunction with an insecticide.

5. A method of influencing the reproduction rate of cockroaches, preferably those of the families Blatta and Periplaneta, wherein an agent as claimed in claim 3 is used in an amount such that the male animals of the species in a family are disoriented in their search for the female animals.

**Revendications**

1. Périplanone A-(±) racémique de la formule I

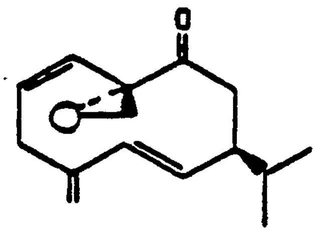

(I)

2. Procédé de préparation de périplanone A-racémique de la formule I selon la revendication 1 caractérisé par le fait qu'en partant de la cyclodécatriénone de la formule II

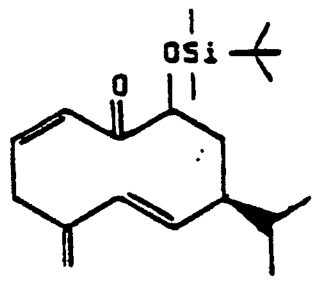

(II)

on produit par oléfinisation le cyclodécatétraène de formule III

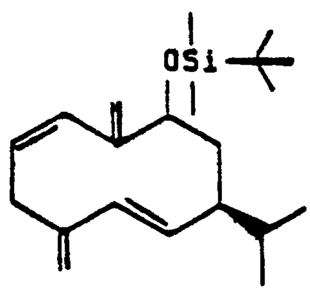

(III)

par séparation du reste t-butyldiméthylsilyle on obtient l'alcool de formule IV qu'on transforme par oxydation en la cyclodécatétraénone de la formule V

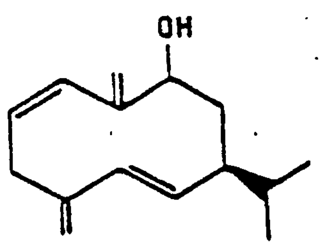

(IV)

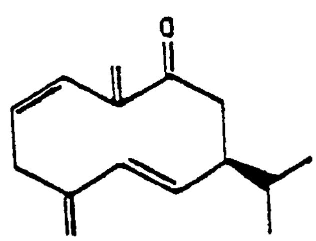

(V)

et qu'on époxyde.

3. Moyen de lutte contre les blattidés, de préférence de la formule des blattes et périplanéta, contenant de la périplanone A de la structure I selon la revendication 1 et éventuellement des additifs et

auxiliaires usuels.

4. Procédé de lutte contre les blattidés, de préférence de la famille des blattes et périplanéta caractérisé par le fait qu'on utilise un moyen selon la revendication 3 en combinaison avec un insecticide.

5. Procédé pour influencer la vitesse de croissance des blattidés, de préférence de la famille des blattes et périplanéta, caractérisé par le fait qu'on applique un moyen selon la revendication 3 en une quantité telle que les animaux mâles du type d'une famille sortent détruits lorsqu'ils se trouvent en présence des animaux femelles.